(19) Europäisches Patentamt
European Patent Office
Office européen des brevets

(11) Veröffentlichungsnummer: **0 440 829 A1**

(12) **EUROPÄISCHE PATENTANMELDUNG**

(21) Anmeldenummer: **90102185.7**

(51) Int. Cl.[5]: **C07D 295/023**, B01J 23/76

(22) Anmeldetag: **03.02.90**

(43) Veröffentlichungstag der Anmeldung: **14.08.91 Patentblatt 91/33**

(84) Benannte Vertragsstaaten: **BE DE ES FR GB IT NL**

(71) Anmelder: **BASF Aktiengesellschaft**
**Carl-Bosch-Strasse 38**
**W-6700 Ludwigshafen(DE)**

(72) Erfinder: **Fischer, Roman, Dr.**
**Deidesheimer Strasse 1**
**W-6704 Mutterstadt(DE)**
Erfinder: **Mueller, Herbert, Dr.**
**Carostrasse 53**
**W-6710 Frankenthal(DE)**
Erfinder: **Voges, Dieter, Dr.**
**Richard-Wagner-Strasse 28**
**W-6800 Mannheim 1(DE)**

(54) **Verfahren zur Herstellung von N-substituierten cyclischen Aminen.**

(57) Verfahren zur Herstellung von N-substituierten cyclischen Aminen der allgemeinen Formel I

A N-R I,

in der

- A eine Alkylen-gruppe oder eine $-(CH_2)_n-[O-(CH_2)_m]_r-$Gruppe, die gegebenenfalls durch Reste R ein- oder mehrfach substituiert sein können, ohne daß die Reste R in einer definierten Verbindung gleich sein müssen und
- R Alkyl, Alkoxyalkyl, gegebenenfalls durch Alkyl oder Alkoxy substituiertes Cycloalkyl, Cycloalkyl-alkyl, Aryl, Arylalkyl,
- n,m unabhängig voneinander 2 bis 8 und
- r 1 bis 3

bedeuten, wobei die $-(CH_2)_n-[O-(CH_2)_m]_r-$Gruppe aus 5 bis 12 Gliedern besteht, durch Umsetzung von primären Aminen der allgemeinen Formel II

$R-NH_2$ (II)

mit einem Diol der allgemeinen Formel III

HO-A-OH (III),

wobei R und A die oben genannten Bedeutungen haben, und Wasserstoff in Gegenwart eines kupferhaltigen Hydrierungs-/Dehydrierungskatalysators bei erhöhten Temperaturen und Drücken, indem man die Umsetzung in Gegenwart einer katalytischen Menge einer basischen Alkalimetall- oder Erdalkalimetallverbindung vornimmt.

EP 0 440 829 A1

## VERFAHREN ZUR HERSTELLUNG VON N-SUBSTITUIERTEN CYCLISCHEN AMINEN

Die vorliegende Erfindung betrifft ein neues und verbessertes Verfahren zur Herstellung von N-substituierten cyclischen Aminen durch Umsetzung von primären Aminen mit Diolen in Gegenwart eines Hydrierungs-/Dehydrierungskatalysators.

Aus der US-A-3 167 551 und der EP-A-691 068 ist bekannt, daß man N-alkylierte heterocyclische Amine aus Diolen mit Ammoniak und anschließender Methylierung mit Formaldehyd oder Methanol unter hydrierenden Bedingungen erhalten kann.

Aus der US-A-3 151 113 ist bekannt, daß man N-alkylierte Morpholine aus dem entsprechenden Diol mit Ammoniak und Methanol unter gleichzeitiger Anwesenheit von Wasserstoff an einem Hydrier/Dehydrierungskatalysator herstellen kann.

N-Alkylmorpholine sind nach der US-A-3 709 881 und der GB-A-1 106 084 ebenfalls aus dem entsprechenden Diol und den entsprechenden Aminen erhältlich. Als Katalysatoren sind u.a. Kupfer, Nickel, Kobalt oder Chrom, deren Mischungen sowie Oxide der Carbonate dieser Metalle empfohlen.

All diese Verfahren lassen in der Anwendungsbreite oder in den erzielten Ausbeuten zu wünschen übrig.

Ferner ist aus der EP-A-137 478 bekannt N-methylierte cyclische Amine (hier als Imine bezeichnet) in der Gasphase an einem Kupferkatalysator herzustellen. Die Raum-Zeit-Ausbeuten liegen jedoch im allgemeinen unter 0,1 kg/l•h.

Der Erfindung lag daher die Aufgabe zugrunde, einen besseren Zugang zu den substituierten cyclischen Aminen zu finden und den Nachteilen der bekannten Verfahren abzuhelfen.

Demgemäß wurde ein neues und verbessertes Verfahren zur Herstellung von N-substituierten cyclischen Aminen der allgemeinen Formel I

A N-R I,

in der

- A eine Alkylen-gruppe oder eine $-(CH_2)_n-[-O-(CH_2)_m]_r$-Gruppe, die gegebenenfalls durch Reste R ein- oder mehrfach substituiert sein können, ohne daß die Reste R in einer definierten Verbindung gleich sein müssen und
- R Alkyl, Alkoxyalkyl, gegebenenfalls durch Alkyl oder Alkoxy substituiertes Cycloalkyl, Cycloalkylalkyl, Aryl oder Arylalkyl,
- n,m unabhängig voneinander 2 bis 8 und
- r 1 bis 3

bedeuten, wobei die $-(CH_2)_n-[O-(CH_2)_m]_r$-Gruppe aus 5 bis 12 Gliedern besteht, durch Umsetzung von primären Aminen der allgemeinen Formel II

$R-NH_2$ (II)

mit einem Diol der allgemeinen Formel III

HO-A-OH (III),

wobei R und A die oben genannten Bedeutungen haben, und Wasserstoff in Gegenwart eines kupferhaltigen Hydrierungs-/Dehydrierungskatalysators bei erhöhten Temperaturen und Drücken gefunden, in dem man die Umsetzung in Gegenwart einer katalytischen Menge einer basischen Alkalimetall- oder Erdalkalimetallverbindung vornimmt.

Die N-substituierten cyclischen Amine der allgemeinen Formel I sind nach folgender Methode erhältlich: Die Umsetzung erfolgt durch Kontakt eines Gemisches aus einem primären Amin II, einem Diol III, Wasserstoff und einer katalytischen Menge einer basischen Alkalimetall- oder Erdalkalimetallverbindung in Gegenwart eines kupferhaltigen Hydrierungs-/Dehydrierungskatalysators nach folgender Reaktionsgleichung:

$$R{-}NH_2 + HO{-}A{-}OH \longrightarrow A \quad N{-}R + 2\,H_2O$$

(II) (III) (I)

Die Reaktion wird in der Flüssigphase diskontinuierlich oder vorzugsweise kontinuierlich bei 150 bis 300 °C und 40 bis 300 bar durchgeführt.

Das Reaktionsgemisch enthält das primäre Amin II zu dem Diol III im Molverhältnis von 0,2:1 bis 5:1, vorzugsweise 0,3:1 bis 3:1, besonders bevorzugt bei 0,5:1 bis 2:1.

Als basische Alkalimetall- oder Erdalkalimetallverbindungen eignen sich sowohl anorganische Alkalimetall- oder Erdalkalimetallbasen, bevorzugt Hydroxide und/oder Oxide der Alkali- und/oder Erdalkalimetalle wie Lithiumhydroxid, Natriumhydroxid, Kaliumhydroxid, Magnesiumhydroxid, Calciumhydroxid, Strontiumhydroxid, Bariumhydroxid, Lithiumoxid, Natriumoxid, Kaliumoxid, Magnesiumoxid, Calciumoxid, Strontiumoxid, Bariumoxid oder deren Gemische, besonders bevorzugt sind Natriumhydroxid und Kaliumhydroxid als auch organische Alkalimetall- oder Erdalkalimetallbasen wie Alkoholate der Alkali- und/oder Erdalkalimetalle wie die $C_1$-$C_4$-Alkoholate der Alkali- und/oder Erdalkalimetalle wie Natriummethylat, Natriumethylat, Kaliummethylat oder Kaliumethylat, besonders bevorzugt Natriummethylat.

Sie werden in katalytischen Mengen von 0,01 bis 10 Gew.% bezogen auf die Summe von primärem Amin II und Diol III, vorzugsweise von 0,01 bis 1 Gew.%, besonders bevorzugt von 0,02 bis 0,2 Gew.% eingesetzt. Zweckmäßig löst oder schlämmt man sie im verwendeten Alkohol auf oder bevorzugt löst man sie in Wasser.

Sowohl das eventuell zusätzlich eingebrachte Wasser als auch das sich bildende Reaktionswasser sollen während der Reaktion im Gemisch verbleiben. Der Wasserstoffpartialdruck während der Umsetzung liegt in der Regel bei 2 bis 300 bar, vorzugsweise bei 50 bis 250 bar, besonders bevorzugt bei 100 bis 250 bar.

Gegebenenfalls verwendet man zusätzlich noch ein Inertgas wie beispielsweise Stickstoff oder Argon, bevorzugt Stickstoff.

Die Flüssigphasenreaktion kann beispielsweise als Suspensions-, Riesel- oder Sumpfreaktion bei Temperaturen von 150 bis 300 °C und Drücken von 50 bis 300 bar, vorzugsweise bei Temperaturen von 180 bis 260 °C und Drücken von 100 bis 250 bar durchgeführt werden. Es empfiehlt sich schwerflüchtige oder feste primäre Amine II oder Diole III in einem inerten Lösungsmittel gelöst zu verwenden, wobei pro Mol II bzw. III zwischen 50 und 200 ml, vorzugsweise 100 bis 150 ml eines inerten Lösungsmittels in aller Regel genügen.

Als inerte Lösungsmittel eignen sich Ether wie Diethylether, Methyl-isopropylether, Methyl-tert.-butylether, Tetrahydrofuran und Dioxan; aliphatische Kohlenwasserstoffe wie n-Pentan, das Pentanisomerengemisch, n-Hexan, das Hexanisomerengemisch, Petrolether und Cyclohexan, aromatische Kohlenwasserstoffe wie Benzol, Toluol, die Xylole und deren Isomerengemisch oder Mischungen dieser Lösungsmittel.

Als Hydrierungs-/Dehydrierungskatalysatoren eignen sich besonders solche, deren katalytische Aktivität hauptsächlich auf Kupfer zurückzuführen ist und deren aktive Masse zu wenigstens 80 Gew.% aus diesem Metall besteht. Besonders bevorzugt sind Kupferkatalysatoren, deren katalytisch aktive Masse zu mehr als 90 Gew.% aus metallischem Kupfer besteht.

Man kann das Kupfer in Form von Pulver, Spänen oder Sklettkatalysator (Raney-Kupfer) einsetzen, jedoch empfiehlt es sich, die Katalysatoren durch Reduktion von Kupferverbindungen wie Kupferacetat, Kupferpropionat und Kupferoxid herzustellen, wobei die Reduktion mit Wassersstoff, Metallhydriden oder organischen Metallverbindungen in situ erfolgen kann.

Beispielsweise eignen sich Katalysatoren aus EP-A-70 512, die sich unter den Reaktionsbedingungen aus Kupferformiat bilden.

Weiterhin geeignete Katalysatoren sind solche, die man nach der DE-A-2 445 303 gewinnt, indem man ein Kupfersalz und ein Aluminiumsalz, z.B. im Molverhältnis Cu:Al von etwa 0,2:1 bis 1:1, enthaltende wäßrige Lösungen mit einem Alkalimetallcarbonat versetzt und das hierbei anfallende basische Cu/Al-Mischcarbonat zu Katalysatorteilchen wie Kugeln oder Strängen formt und anschließend bei Temperaturen von 200 bis 600 °C trocknet. Diese Teilchen bilden unter den Reaktionsbedingungen ein poröses Gerüst aus Aluminiumoxid und -oxidhydraten mit einer entsprechenden großen Oberflächenschicht aus metallischem Kupfer.

Es können auch kupferhaltige Trägerkatalysatoren verwendet werden, die man durch Tränken von Trägermaterialien wie Bimsstein, Diatomeenerde, Kieselgel oder Aluminiumoxid mit Kupfersalz-Lösungen behandelt, trocknet und anschließend z.B. mit Wasserstoff reduziert.

Neben der Hauptkomponente Kupfer können die Katalysatoren auch noch untergeordnete Mengen anderer Bestandteile z.B. Kobalt und Nickel enthalten.

Bei der diskontinuierlichen Betriebsweise verwendet man die Katalysatoren vorzugsweise in Form feinteiliger Suspensionen. Bei den kontinuierlichen Verfahren empfehlen sich Festbettkontakte, die durch Rieselfahrweise oder bevorzugt durch Sumpffahrweise bei der die gesamte Katalysatorschicht zusammenhängend mit Flüssigkeit bedeckt sein sollte, betrieben werden.

Die Menge der Katalysatoren entspricht beim diskontinuierlichen Suspensionsverfahren vorzugsweise 10 bis 150, besonders 20 bis 70 g Kupfer pro kg Reaktionsgemisch und für den kontinuierlichen Betrieb am Festbettkatalysator vorzugsweise im Bereich von 100 bis 500, besonders 200 bis 400 g Kupfer pro kg Reaktionsgemisch pro Stunde.

Man kann bei dem diskontinuierlichen Betrieb, das Diol III mit dem darin suspendierten Katalysator und einer gelösten oder suspendierten anorganischen Base in Wasser vorlegen und das Amin II nach Maßgabe fortschreiten-der Reaktion bei der Reaktionstemperatur unter den gewählten Bedingungen allmählich hinzuzugeben. Insbesondere beim Einsatz der Reaktanden in äquimolaren Mengen kann man die Komponenten Diol, Amin, Wasser und Base auch gemeinsam vorlegen.

Arbeitet man kontinuierlich, so führt man das Diol III und das Amin II im gewählten Molverhältnis über ein Kupferkatalysator-Festbett, wobei die anorganische Base und das Wasser in einer oder beiden dieser Komponenten gelöst, bzw. suspendiert sein können.

Nach der Umsetzung werden die entstandenen Produkte durch übliche Verfahren, z.B. durch Destillation oder Extraktion aus dem Reaktionsgemisch isoliert; nichtumgesetzte Ausgangsstoffe werden gegebenenfalls in die Umsetzung zurückgeführt.

Die Substituenten in den Formeln I bis III haben folgende Bedeutung:

A

- eine Alkylen-gruppe, wie eine $C_4$-$C_{12}$-Alkylen-gruppe, z.B. $-(CH_2)_4-$, $-(CH_2)_5-$, $-(CH_2)_6-$, $-(CH_2)_7-$, $-(CH_2)_8-$, $(CH_2)_9-$, $-(CH_2)_{10}-$, $-(CH_2)_{11}-$ und $-(CH_2)_{12}-$, von denen die Alkylen-gruppen mit 4 bis 8 bevorzugt sind,
- eine $-(CH_2)_n-[O-(CH_2)_m]_r$-Gruppe, in der n und m unabhängig voneinander 2 bis 8 und r 1 bis 3 bedeuten, wobei die $-(CH_2)_n-[O-(CH_2)_m]_r$-Gruppe aus 5 bis 12 Gliedern besteht, vorzugsweise n und m unabhängig voneinander 2 bis 4 und r 1 bis 2, wie $-(CH_2)_2-O-(CH_2)_2-$, $-(CH_2)_3-O-(CH_2)_2-$, $-(CH_2)_3-O-(CH_2)_3-$, $-(CH_2)_4-O-(CH_2)_2-$, $-(CH_2)_4-O-(CH_2)_3-$, $-(CH_2)_4-O-(CH_2)_4-$ und $-(CH_2)_2-O-(CH_2)_2-O-(CH_2)_2-$.
- A kann durch einen oder mehrere der nachfolgenden Reste R substituiert sein, wobei die Reste R in einer definierten Verbindung nicht gleich sein müssen. Bevorzugt sind 1 bis 6 Reste R, besonders bevorzugt 1 bis 4.

R

- Alkyl, wie unverzweigtes oder verzweigtes $C_1$- bis $C_{30}$-Alkyl, bevorzugt unverzweigtes oder verzweigtes $C_1$- bis $C_{20}$-Alkyl, besonders bevorzugt unverzweigtes oder verzweigtes $C_1$- bis $C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- Alkoxy-alkyl, bevorzugt $C_2$- bis $C_{20}$-Alkoxy-alkyl, wie Methoxymethyl, Ethoxypropyl und Butoxydecyl,
- Cycloalkyl, bevorzugt $C_3$- bis $C_{20}$-Cycloalkyl, besonders bevorzugt $C_3$- bis $C_8$-Cycloalkyl, wie Cyclopropyl, Cyclobutyl, Cyclopentyl, Cyclohexyl, Cycloheptyl und Cyclooctyl,
- Alkyl-cycloalkyl, bevorzugt $C_4$- bis $C_{20}$-Alkyl-cycloalkyl, wie Methyl-cyclopropyl, Isopropyl-cyclohexyl und Dodecyl-cyclohexyl,
- Cycloalkyl-alkyl, bevorzugt $C_4$- bis $C_{20}$-Cycloalkyl-alkyl, wie Cyclopropyl-methyl, Cyclopentyl-methyl, Cyclohexyl-methyl, Cyclopropyl-ethyl, Cyclopentyl-ethyl und Cyclohexyl-ethyl,
- Aryl, wie Phenyl, 1-Naphthyl, 2-Naphthyl, 1-Anthyl, 2-Anthyl und 9-Anthzyl, bevorzugt Phenyl,
- Arylalkyl, bevorzugt $C_7$- bis $C_{20}$-Arylalkyl, besonders bevorzugt $C_7$- bis $C_{10}$-Phenylalkyl, wie Benzyl, Phenethyl oder Phenyldecyl.

Endprodukte der Formel I sind beispielsweise:
N-Methylpyrrolidin, N-Ethylpyrrolidin, N-Methylpiperidin, N-Ethylpiperidin, N-Methylmorpholin, 1-Methyl-1-azacycloundecan und N-n-Butylmorpholin.

Ausgangsstoffe der Formel II sind beispielsweise:
Methylamin, Ethylamin, n-Propylamin, iso-Propylamin, n-Butylamin, Isobutylamin, n-Dodecylamin, Anilin und p-Toluidin.

Ausgangsstoffe der Formel III sind beispielsweise:
Butan-1,4-diol,
3-Methylpentan-1,5-diol,
Diethylenglykol (3-Oxapentan-1,5-diol),
Dibutylenglykol (5-Oxanonan-1,9-diol)
Triethylenglykol (3,5-Dioxaheptan-1,7-diol),
1,3-Dipropylenglykol (4-Oxaheptan-1, -diol),
Dipropylenglykol (1,5-Dimethyl-3-Oxapentan-1,5-diol),

5-Oxanonan-1,9-diol und
Hexan-2,5-diol.

Allgemein eignen sich als Diole III solche mit primären oder sekundären Hydroxylgruppen besonders solche mit primären Hydroxylgruppen.

Die nach dem erfindungsgemäßen Verfahren herstellbaren sekundären Amine der allgemeinen Formel I sind z.B. Reaktionspartner oder spezielle Säureacceptoren in Synthesis von Pharmazeutika, von Wirkstoffen für den Pflanzenschutz, von Farbstoffen und als basische Katalysatoren z.B. für die Herstellung von Polyurethanen.

Die folgenden Beispiele dienen zur weiteren Erläuterung des erfindungsgemäßen Verfahrens ohne die Erfindung einzuschränken.

Beispiele

Herstellung des Katalysators

Aus einer wäßrigen Lösung von Kupfernitrat und Aluminiumnitrat wurde mit Natriumhydrogencarbonat (Molverhältnis Kupfer zu Aluminium 1:1,2) ein Mischcarbonat nach Beispiel 1 der DE-A-24 45 303 gefällt, zu Zylindern von 3 mm Länge und 3 mm Durchmesser geformt, getrocknet und anschließend in das Reaktionsrohr gefüllt, darin bei 180 bis 200 °C mit 300 ml/Stunde mit einer 0,01gew.%igen methanolischen Natriummethylatlösung unter 50 bar Wasserstoffdruck behandelt.

Beispiel 1

Herstellung von N-Methylpiperidin

Durch einen Rohrreaktor (Durchmesser = 3,2 cm und 125 cm Höhe), der eine Schüttung von 700 ml des vorstehend hergestellten Katalysators enthielt, wurden stündlich 280 ml eines Gemisches, das aus 1040 g Pentan-1,5-diol und 310 g Methylamin und 10 g 45 %iger wäßriger Kaliumhydroxidlösung besteht bei 245 °C, 250 bar Gesamtdruck und 120 bar Wasserstoffpartialdruck geleitet. Man erhält N-Methylpiperidin in 95 %iger Ausbeute.

Beispiel 2

Herstellung von N-(n-Butyl)-pyrrolidin

Analog Beispiel 1 wurden 11,7 kg Butan-1,4-diol mit 7,3 kg n-Butylamin umgesetzt. Man erhält N-(n-Butyl)-pyrrolidin in 96 %iger Ausbeute.

Beispiel 3

Herstellung von N-Methylmorpholin

Analog Beispiel 1 wurden 1060 g Diethylenglykol mit 310 g Methylamin umgesetzt. Man erhielt N-Methylmorpholin in 98 %iger Ausbeute.

**Patentansprüche**

**1.** Verfahren zur Herstellung von N-substituierten cyclischen Aminen der allgemeinen Formel I

A N-R I,

in der

A eine Alkylen-gruppe oder eine $-(CH_2)_n-[O-(CH_2)_m]_r$-Gruppe, die gegebenenfalls durch Reste R ein- oder mehrfach substituiert sein können, ohne daß die Reste R in einer definierten Verbindung gleich sein müssen und

R Alkyl, Alkoxyalkyl, gegebenenfalls durch Alkyl oder Alkoxy substituiertes Cycloalkyl, Cycloalkyl-alkyl, Aryl oder Arylalkyl,

n,m unabhängig voneinander 2 bis 8 und

r 1 bis 3

bedeuten, wobei die $-(CH_2)_n-[O-(CH_2)_m]_r-$Gruppe aus 5 bis 12 Gliedern besteht, durch Umsetzung von primären Aminen der allgemeinen Formel II

$R-NH_2$ (II)

mit einem Diol der allgemeinen Formel III

HO-A-OH (III) ,

wobei R und A die oben genannten Bedeutungen haben, und Wasserstoff in Gegenwart eines kupferhaltigen Hydrierungs-/Dehydrierungskatalysators bei erhöhten Temperaturen und Drücken, dadurch gekennzeichnet, daß man die Umsetzung in Gegenwart einer katalytischen Menge einer basischen Alkalimetall- oder Erdalkalimetallverbindung vornimmt.

**2.** Verfahren nach Anspruch 1, dadurch gekennzeichnet, daß man als basische Verbindungen 0,01 bis 10 Gew.% zumindest eines Hydroxides und/oder Oxides und/oder $C_1$-$C_4$-Alkoholates der Alkali- und/oder Erdalkalimetalle, bezogen auf die Summe von primärem Amin II und Diol III verwendet.

**3.** Verfahren nach Anspruch 1 oder 2, dadurch gekennzeichnet, daß man die Umsetzung in einem Molverhältnis von primärem Amin II zum Diol III von 0,2:1 bis 5:1 vornimmt.

**4.** Verfahren nach Anspruch 1 oder 2, dadurch gekennzeichnt, daß man die Umsetzung in einem Molverhältnis von primärem Amin II zum Diol III von 0,5:1 bis 2:1 vornimmt.

**5.** Verfahren nach den Ansprüchen 1 bis 4, dadurch gekennzeichnet, daß man als Hydrierungs-/Dehydrierungskatalysatoren, solche verwendet, die als katalytisch wirksamen Metallbestandteil im wesentlichen Kupfer enthalten.

**6.** Verfahren nach den Ansprüchen 1 bis 5, dadurch gekennzeichnet, daß man die Umsetzung ohne Entfernung des Reaktionswassers durchführt.

**7.** Verfahren nach den Ansprüchen 1 bis 6, dadurch gekennzeichnet, daß man die Umsetzung bei Temperaturen von 150 bis 300 °C vornimmt.

**8.** Verfahren nach den Ansprüchen 1 bis 7, dadurch gekennzeichnet, daß man die Umsetzung bei Drücken von 50 bis 300 bar vornimmt.

**9.** Verfahren nach den Ansprüchen 1 bis 8, dadurch gekennzeichnet, daß man die Umsetzung als Flüssigphasenreaktion vornimmt.

Europäisches Patentamt

# EUROPÄISCHER RECHERCHENBERICHT

Nummer der Anmeldung

EP 90 10 2185

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 137 478 (BASF AKTIENGESELLSCHAFT) * ganzes Dokument * --- | 1 | C 07 D 295/023<br>B 01 J 23/78 |
| Y | EP-A-0 070 397 (BASF AKTIENGESELLSCHAFT) * Seite 2, Zeile 14 - Seite 4, Zeile 17 * --- | 1 | |
| Y,D | EP-A-0 070 512 (BASF AKTIENGESELLSCHAFT) * Seite 5, Zeile 4 - Seite 8, Zeile 5 * --- | 1 | |
| Y,D | DE-A-2 445 303 (BASF AKTIENGESELLSCHAFT) * ganzes Dokument * --- | 1 | |
| Y,D | US-A-3 709 881 (G.H. WARNER) * ganzes Dokument * ----- | 1 | |

RECHERCHIERTE SACHGEBIETE (Int. Cl.5)

C 07 D 295/00
B 01 J 23/00

Der vorliegende Recherchenbericht wurde für alle Patentansprüche erstellt

| Recherchenort | Abschlußdatum der Recherche | Prüfer |
|---|---|---|
| BERLIN | 17-09-1990 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)